(19) Europäisches Patentamt
European Patent Office
Office européen des brevets

(11) Numéro de publication: **0 099 438**
**A1**

(12) # DEMANDE DE BREVET EUROPEEN

(21) Numéro de dépôt: 82401345.2

(22) Date de dépôt: 20.07.82

(51) Int. Cl.³: C 07 D 253/06
C 07 D 401/12, A 61 K 31/53

(43) Date de publication de la demande:
01.02.84 Bulletin 84/5

(84) Etats contractants désignés:
AT BE CH DE GB IT LI LU NL SE

(71) Demandeur: PIERRE FABRE S.A.
125, rue de la Faisanderie
F-75116 Paris(FR)

(72) Inventeur: Pitet, Guy
3, rue de l'Aubisque
F-31000 Toulouse(FR)

(72) Inventeur: Couret, Françoise
Chemin de Thil
F-31450 Corransac(FR)

(74) Mandataire: Doat, Jean Pierre
17, Avenue Jean Moulin
F-81106 Castres Cedex(FR)

(54) **Diaryl as-triazines fonctionnalisées en 2 utiles en thérapeutique.**

(57) La présente invention concerne l'utilisation en thérapeutique de nouveaux dérivés triaziniques doués de propriétés antalgiques et anti-agrégantes utilisables dans le traitement d'algies, migraines.

Les nouveaux dérivés ont pour formule générale:

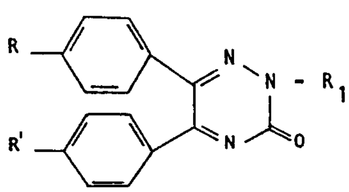

R et R' identiques ou différents représentent un hydrogène, alcoyl, alcoxy, un dialcoylamino, un halogène.

Le groupe $R_1$ est un groupement alcoyle fonctionnalisé ou un groupement de type hétérocyclique, $R_1$ représente plus particulièrement un groupe morpholino alcoyl, diméthylamino éthyl, alcoxy alcoyl, alcenoxy alcoyl, hydroxy alcoyl, acétamido, halogéno alcoyl, diméthylamino alcoyl, N-méthyl pipérazinyl alcoyl, diéthylmalonyl-2, alcenyl amino alcoyl, nicotinoyloxy éthyl-2, dihydroxyalcoyl, à l'exclusion des composés dans lesquels $R_1$ représente un groupe alcoyl, alcenyl ou une fonction cétonique.

EP 0 099 438 A1

Diaryl as-triazines fonctionnalisées en 2 utiles en thérapeutique.

La présente invention, réalisée au Centre de Recherches Pierre Fabre, avec la collaboration technique de Mme Irène MOURET, a pour objet des composés chimiques nouveaux, leur procédé de préparation et leur application en tant que médicaments. Ils sont utiles notamment dans le traitement des algies. L'invention vise également les compositions pharmaceutiques contenant ces principes actifs.

Les composés chimiques selon l'invention sont de nouveaux dérivés triaziniques de formule :

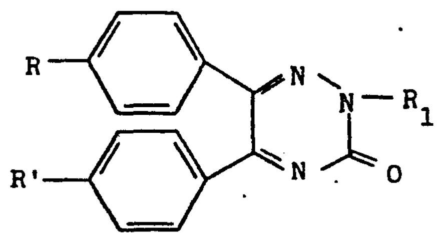

dans laquelle :

R et R' identiques ou différents représentent un hydrogène, alcoyl, alcoxy, un dialcoyl amino, un halogène.

Le groupe $R_1$ est un groupement alcoyle fonctionnalisé ou un groupement de type hétérocyclique, $R_1$ représente plus particulièrement un groupe morpholino alcoyl, diméthylamino éthyl, alcoxy alcoyl, alcenoxy alcoyl, hydroxy alcoyl, acétamido, halogéno alcoyl, diméthylamino alcoyl, N méthyl piperazino alcoyl, diéthyl malonyl-2, alcenyl-amino-alcoyl, nicotinoyl-oxy-éthyl-2, dihydroxy-alcoyl.

Les composés objet de l'invention peuvent être obtenus selon le schéma général :

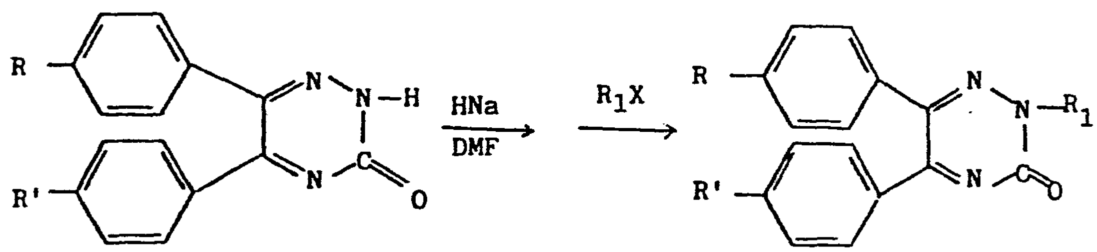


Les intermédiaires triaziniques non substitués en 2 ont été préparés antérieurement par la demanderesse.

L'obtention du cycle triazinique à partir d'$\alpha$ dicétones est rappelée :

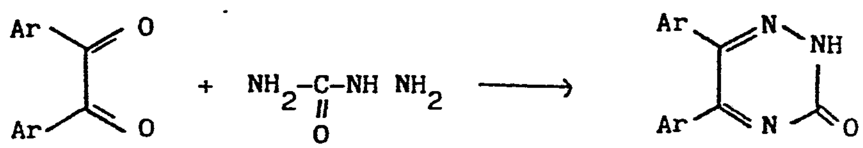

Ar = groupe aryle substitué ou non.

Les diaryl $\alpha$ dicétones sont accessibles par différentes méthodes selon G. Pitet, H. Cousse, G. Mouzin. - Boll. Chim. Farm. 1980, 119, p. 469-482.

Mode opératoire

Les dérivés de formule générale :

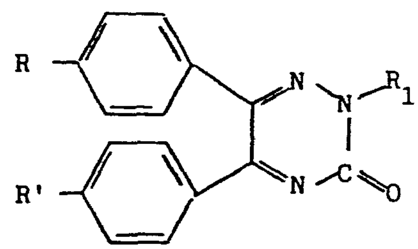

sont obtenus en traitant une mole d'oxy-3 di(aryl)-5-6 as triazine en solution dans le DMF, par une mole d'hydrure de sodium, puis par une quantité stoechiométrique de réactif halogéné.

Les sels d'amine sont obtenus en traitant la base correspondante par une solution éthérée de l'acide correspondant.

Les composés cités ne sont pas à notre connaissance décrits dans la littérature.

(Les CCM sont effectuées sur plaques de silice Merck GF 254, révélateur U.V. $\lambda$: 254 nm).

A titre d'exemple, l'obtention d'un composé selon la présente invention est décrite ci-après :

Obtention de l'hydroxyéthyl-2 di(paradiméthyl amino phényl)-5-6 as triazine (ST 817)

A une suspension dans le diméthylformamide de 1,44 g soit 3/100 mole d'hydrure de sodium en dispersion à 50 % dans l'huile, on ajoute une suspension équimoléculaire d'hydroxy-3 di(paradiméthyl amino phényl)-5-6 as triazine dans le diméthylformamide. On observe un dégagement gazeux de 680 ml (théorie : 672 ml). Ajouter une quantité stoechiométrique de de Cl-CH2-CH2OH. Le milieu réactionnel est agité 12 heures à température ambiante puis concentré à sec sous vide et repris à l'eau. Un produit solide rouge est essoré sur fritté, séché et recristallisé dans 20 volumes de butanol normal.

On recueille finalement 5,3 g de produit sec, soit un rendement de 47 %.

P.F. : 222°C.

Le produit est homogène en ccm : Rf : 0,07 (toluène - dioxanne $NEt_3$ 8/1, 8/1,5/0,5).

Il présente en infra-rouge des bandes $\nu$ C=O à 1660 $cm^{-1}$ et $\nu$ OH à 3550 $cm^{-1}$.

Microanalyse : C % : 66,48 H % : 6,57 N % : 18,04.

En utilisant le mode opératoire précédemment décrit, il a été obtenu d'autres composés chimiques, dont quelques exemples non limitatifs sont cités ci-après :

Exemple 1 : Oxo-3 morpholino éthyl-2 di(paraméthoxyphényl)-5-6 as triazine (ST 728)

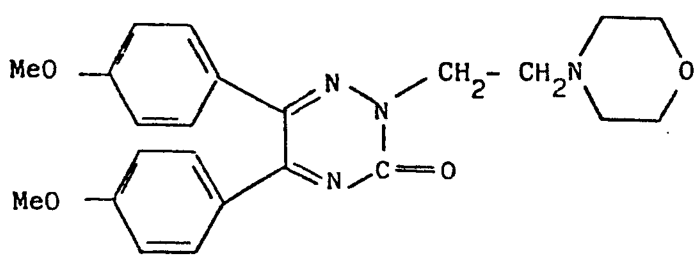

- Cristaux jaunes
- Soluble dans les acides, les alcools, le benzène, le chlorure de méthylène et le chloroforme
- Insoluble dans l'eau, les bases diluées et l'éther
- Point de fusion : 128 ± 2°C
- C.C.M. : benzène - dioxanne - $NEt_3$ (8/1,5/0,5) - Rf : 0,31.
- I.R. : $\nu$C = 0 1650 - 1660 $cm^{-1}$.

Exemple 2 : Oxo-3 βméthyl-N-N-diméthylamino-2 éthyl di(paraméthoxyphényl) -5-6 as triazine (ST 766)

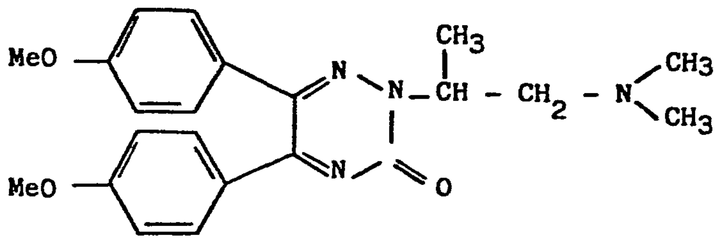

- Cristaux jaunes
- Soluble dans les acides dilués, les alcools, le chlorure de méthylène et le chloroforme
- Insoluble dans l'eau, les bases diluées, le benzène et l'éther.
- Point de fusion : 156°C.
- C.C.M. : toluène - dioxanne - $NEt_3$ (8/1,5/0,5)

  Rf : 0,24
- I.R. : $\nu_{C = 0}$ 1655$cm^{-1}$.

Exemple 3 : Oxo-3 vinyloxyéthyl-2 di(paraméthoxyphényl)-5-6 as triazine (ST 777)

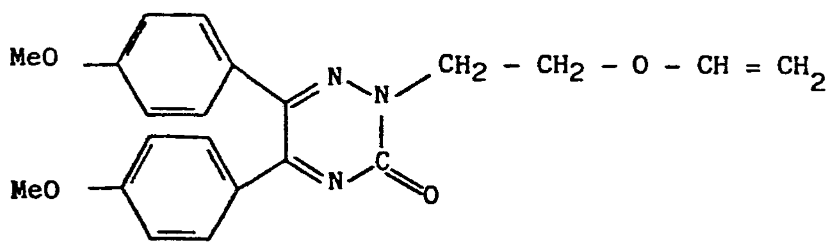

- Cristaux jaunes
- Soluble dans les alcools, le benzène, le chlorure de méthylène et le chloroforme
- Insoluble dans l'eau, les bases et les acides dilués et l'éther
- Point de fusion : 83 ± 2°C.
- C.C.M. : benzène - AcOEt (6/4)

  Rf : 0,37
- I.R.. : $\nu_{C = 0}$ 1650-1660 $cm^{-1}$

Exemple 4 : Oxo-3 hydroxyéthyl-2 di(paraméthoxyphényl)-5-6 as triazine (ST 791)

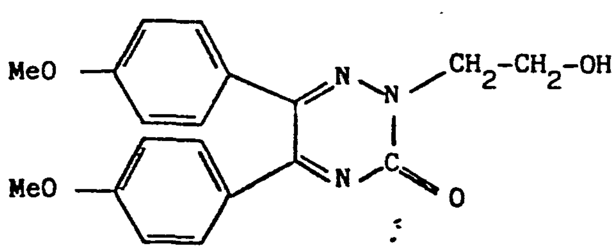

- Cristaux jaunes
- Soluble dans les alcools, le benzène, le chlorure de méthylène et le chloroforme.
- Insoluble dans l'eau, les bases et les acides dilués et l'éther.
- Point de fusion : 137 ± 1°C.
- C.C.M. : MeOH - $CHCl_3$ (10/50)

  Rf : 0,7
- I.R. : $\nu_{C=O}$ : 1650-1660 cm$^{-1}$

  $\nu_{OH}$ : 3380 cm$^{-1}$.

<u>Exemple 5</u> : Oxo-3 acétamido-2 di(paradiméthyl amino phényl)-5-6 as triazine (ST 812)

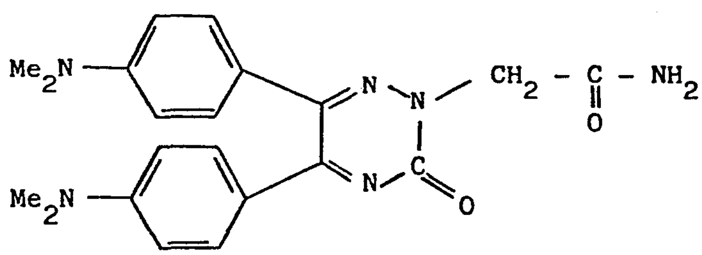

- Cristaux oranges
- Soluble dans les acides dilués et le chlorure de méthylène
- Insoluble dans l'eau, les bases diluées, les alcools, le benzène et l'éther,
- Point de fusion : 190 ± 1°C.
- C.C.M. : ne migre pas dans le benzène - dioxanne - $NEt_3$ (8/1,5/0,5 $CHCl_3$-acétone (7/3)

  Rf : 0,09
- I.R. : $\nu_{C=O}$ : 1640 cm$^{-1}$

  $\nu_{C(=O)-NH_2}$ : 1695 cm$^{-1}$

  $\nu_{NH_2}$ : 3180 - 3280 cm$^{-1}$

<u>Exemple 6</u> : Oxo-3 hydroxyéthyl-2 di(paradimétnyl amino phényl)-5-6 as triazine (ST 817)

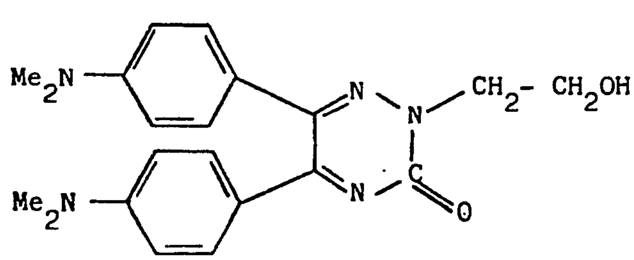

- Cristaux orangés
- Soluble dans les acides dilués, les alcools, le chlorure de méthylène et le chloroforme
- Insoluble dans les bases diluées aqueuses, l'eau, le benzène et l'éther
- Point de fusion : 222 ± 2°C.
- CCM : toluène - dioxanne - $NEt_3$ (8/1,5/0,5)

  Rf : 0,07
- I.R. : $\nu$C = O : 1640 $cm^{-1}$ ; $\nu$OH : 3350 $cm^{-1}$ (bande large).

Exemple 7 : Oxo-3 diéthylmalonyl-2 di(paraméthoxy phényl)-5-6 as triazine (ST 824)

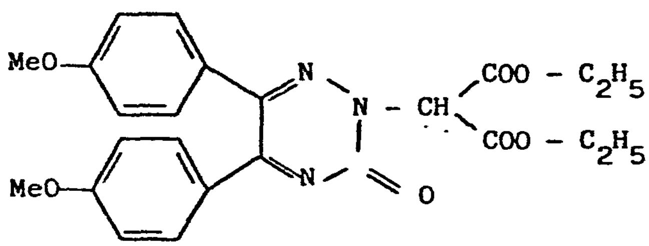

- Cristaux jaunes
- Soluble dans les alcools, le benzène, le chlorure de méthylène et le chloroforme
- Insoluble dans l'eau, les bases et les acides dilués et l'éther
- Point de fusion : 99°C.
- C.C.M. : toluène - AcOEt (7/3)

  Rf : 0,37
- I.R. : $\nu$C = O : 1670 $cm^{-1}$

  $\nu$COO : 1700 $cm^{-1}$.

Exemple 8 : Oxalate de l'oxo-3 $\gamma$allylamino propyl-2 di(paraméthoxy phényl)-5-6 as triazine (ST 876)

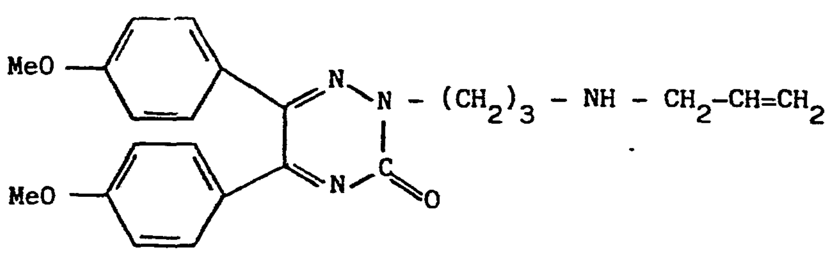

$,C_2H_2O_4 + \frac{1}{2}\ H_2O$

- Cristaux jaunes
- Soluble dans l'eau, les alcools, et le chlorure de méthylène
- Insoluble dans le benzène, l'éther et le chloroforme
- Point de fusion : 160°C

- C.C.M. : toluène - dioxanne - $NEt_3$ (8/1,5/0,5)
  Rf : 0,13
- I.R. : $\nu$ C = O : 1650 $cm^{-1}$ - $\nu$ OH : 3400 $cm^{-1}$.

Exemple 9 : Dichlorhydrate de l'oxo-3 N méthyl piperazinyl-3' propyl -2 di(paraméthoxyphényl)-5-6 as triazine (ST 881)

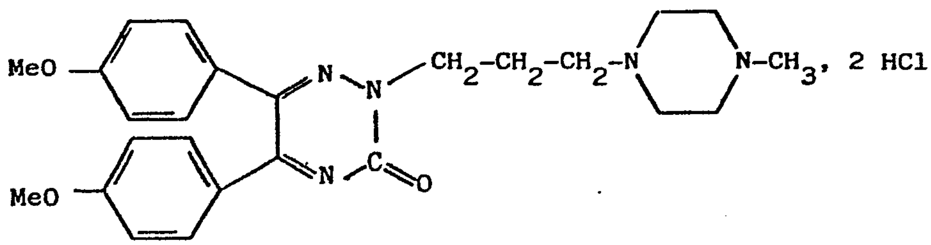

- Cristaux jaunes
- Soluble dans l'eau, les acides dilués et les alcools
- Insoluble dans le benzène, le chlorure de méthylène et le chloroforme
- Point de fusion : 200°C
- C.C.M. : benzène - dioxanne - $NEt_3$ (8/1,5/0,5)
  Rf : 0,04
- I.R. : $\nu$ C = O : 1670 $cm^{-1}$
  absorption "chlorhydrate" entre 2200 et 2700 $cm^{-1}$

Exemple 10 : Oxo-3 $\gamma$-chloropropyl-2 di(paradiméthylaminophényl) 5-6 as triazine (ST 882)

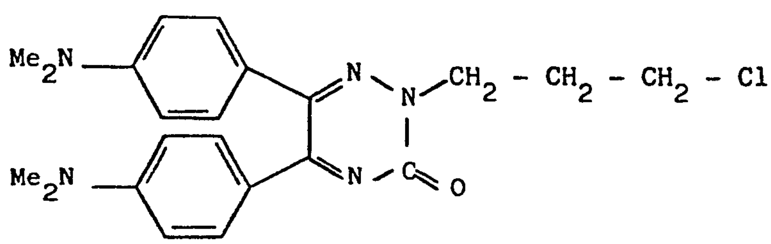

- Cristaux jaunes
- Soluble dans les acides dilués aqueux, les alcools, le benzène, le chlorure de méthylène et le chloroforme.
- Insoluble dans l'eau et les bases diluées, et l'éther
- Point de fusion : 182°C
- C.C.M. : toluène - dioxanne - $NEt_3$ (8/1,5/0,5)
  Rf : 0,32
- I.R. : $\nu$ C = O : 1660 $cm^{-1}$

Exemple 11 : Oxo-3 nicotinoyloxyéthyl-2 di(paradiméthylaminophényl) 5-6 as triazine (ST 883)

(Ce composé est obtenu par action de l'anhydride nicotinique sur ST 817).

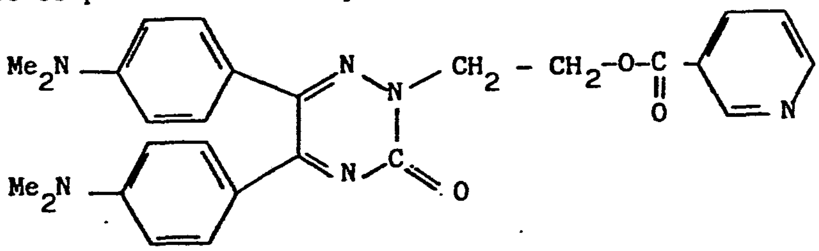

- Cristaux orangés
- Soluble dans les acides dilués, les alcools, le benzène, le chloroforme et le chlorure de méthylène
- Insoluble dans l'eau et les bases diluées, l'éther
- Point de fusion : 160°C.
- C.C.M. : $CHCl_3$ - acétone (7/3)

  Rf : 0,34
- I.R. : $\nu_{C=O}$ : 1660 $cm^{-1}$ - $\nu_{C(=O)-O}$ : 1720 $cm^{-1}$.

Exemple 12 : Oxo-3 (dihydroxy 2'-3' propyl-2 di (paradiméthyl amino phényl)-5-6 as triazine (ST 886)

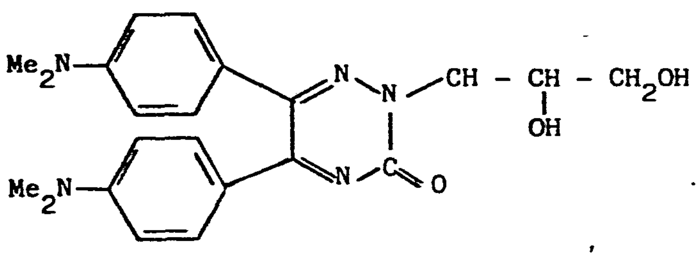

- Cristaux orangés
- Soluble dans les acides dilués aqueux, les alcools, le benzène, le chlorure de méthylène et le chloroforme
- Insoluble dans l'eau et les bases diluées, l'éther
- Point de fusion : 152°C puis 178°C
- C.C.M. ne migre pas dans le benzène - dioxanne - $NEt_3$ (8/1,5/0,5)
- I.R. : $\nu_{C=O}$ : 1640 $cm^{-1}$ - $\nu_{OH}$ (bande large 3300-3500 $cm^{-1}$).

**Exemple 13** : Oxo-3 chloropropyl-2 di(paraméthoxyphényl)-5-6 as triazine (ST 938)

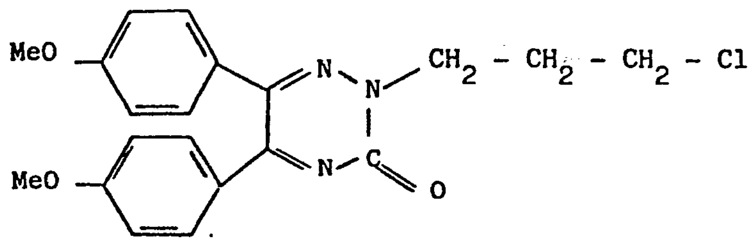

- Cristaux jaunes
- Soluble dans les alcools, le benzène, le chlorure de méthylène et le chloroforme,
- Insoluble dans l'eau, les bases et les acides dilués et l'éther
- Point de fusion : 112°C
- C.C.M. : toluène - ACOEt (7/3)

  Rf : 0,27
- I.R. : $\nu$ C = O : 1650 $cm^{-1}$.

EXPERIMENTATIONS PHARMACOLOGIQUES

Les expérimentations pharmacologiques ont été effectuées dans le laboratoire de Pharmacologie du Centre de Recherches Pierre FABRE dirigé par le Docteur Henri LAURESSERGUES avec la participation de Marie CHARVERON et Antoine STENGER.

a) Toxicologie

L'étude de la toxicité a été effectuée chez la souris conventionnelle pesant environ 20 grammes.

Les substances ont été administrées par voie orale. Les doses létales 50 sont exprimées en mg/kg et ont été calculées selon la méthode de MILLER et TAINTER, Proc. Soc. Exper. Biol. Med. 1944, 57, 261.

Résultats :

| Produits | $DL_{50}$ voie orale |
|---|---|
| ST 728 | >1000 |
| ST 766 | 750 |
| ST 777 | >1000 |
| ST 791 | >1000 |
| ST 812 | >1000 |
| ST 817 | >1000 |
| ST 824 | >1000 |
| ST 876 | 750 |
| ST 881 | >1000 |
| ST 882 | >1000 |
| ST 883 | >1000 |
| ST 886 | >1000 |
| ST 938 | >1000 |

b) Propriétés antalgiques

L'activité sur le test des contorsions à la phényl benzoquinone (PBQ) selon SIEGMUND et Coll., J. Pharm. Exptl. Ther. 1957, 119, 453, a été déterminée après administration du produit per os ; la dose est exprimée en mg/kg chez la souris 30 minutes avant l'injection intra-péritonéale de l'agent algogène.

Les résultats sont exprimés en pourcentage de variation du nombre de contorsions. Pour les molécules les plus actives nous avons déterminé la $DE_{50}$, les résultats sont répertoriés dans le tableau suivant :

| Composés | $DE_{50}$ mg/kg per os |
|---|---|
| ST 766 | 11 |
| ST 777 | 10 |
| ST 791 | 9 |
| ST 812 | 12 |
| ST 817 | 1,5 |
| ST 881 | 6,7 |
| ST 882 | 4,6 |
| ST 883 | 3,1 |
| ST 886 | 5,6 |

c) Propriétés antiagrégantes (in vitro)

Les produits sont dissous dans le PEG 400, puis additionnés au P.R.P. de LAPIN puis incubés une minute avant l'addition d'agent agrégant (acide arachidonique 2 x $10^{-4}$M). Le produit le plus actif est le ST 938.

5 APPLICATIONS THERAPEUTIQUES

Compte-tenu de la parfaite tolérance chez l'animal et des propriétés pharmacologiques ces dérivés et plus particulièrement les produits suivants : ST 817, ST 882, ST 883, ST 886 et ST 881, sont utiles dans le traitement des algies de toutes origines (dentaire, musculaire, rhumatismale).

REVENDICATIONS

1) A titre de composés chimiques nouveaux, les dérivés de formule générale :

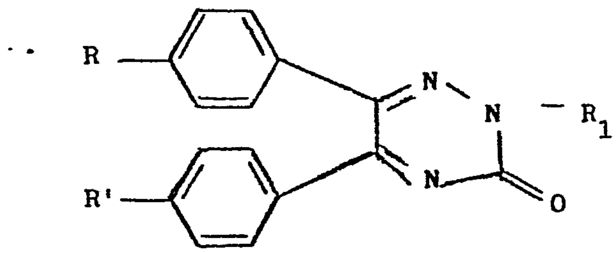

R et R' identiques ou différents représentent : alcoyl, alcoxy, un dialcoylamino, un halogène.

Le groupe $R_1$ est un groupement alcoyle fonctionnalisé ou un groupement de type hétérocyclique, plus particulièrement un groupe morpholino alcoyl, alcoxy alcoyl, alcenoxy alcoyl, N-méthyl pipérazinyl alcoyl, nicotinoyloxy éthyl-2, dihydroxyalcoyl, à l'exclusion des composés dans lesquels $R_1$ représente un groupe alcoyl, alcenyl ou céto-alcoyle.

2) Les composés chimiques selon la revendication 1 et plus particulièrement:

- oxo-3 morpholinoéthyl-2 di(paraméthoxyphényl)-5-6 as triazine (ST 728)
- oxo-3 vinyloxy éthyl-2 di(paraméthoxyphényl)-5-6 as triazine (ST 777)
- oxo-3 hydroxyéthyl-2 di(paraméthoxyphényl)-5-6 as triazine (ST 791)
- oxo-3 acétamido-2 di(paradiméthylaminophényl)-5-6 as triazine (ST 812)
- Oxo-3 hydroxyéthyl-2 di (paradiméthylaminophényl)-5-6 as triazine (ST 817)
- Oxo-3 diéthylmalonyl-2 di (paraméthoxy phényl)-5-6 as triazine (ST 824)
- Oxalate de l'oxo-3 γallylaminopropyl-2 di(paraméthoxy phényl)-5-6 as triazine (ST 876)
- Dichlorhydrate de l'oxo-3 N-méthyl pipérazinyl-3' propyl-2 di (paraméthoxyphényl)-5-6 as triazine (ST 881)
- Oxo-3 γ-chloropropyl-2 di (paradiméthylaminophényl)-5-6 as triazine (ST 882)
- Oxo-3 nicotinoyloxy éthyl-2 di(paradiméthylaminophényl)-5-6 as triazine (ST 883)
- Oxo-3 (dihydroxy-2'-3' propyl)-2 di(paradiméthylamino phényl)-5-6 as triazine (ST 886)
- Oxo-3 γ chloropropyl-2 di(paraméthoxyphényl)-5-6 as triazine (ST 938).

3) Le procédé d'obtention des composés selon les revendications 1 et 2 qui consiste à traiter les triazines de formule :

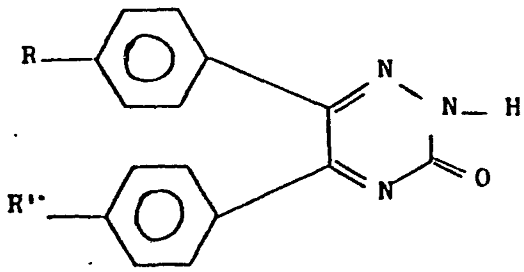

en présence d'agent sodant par un groupe $R_1X$

$R_1$ défini dans la revendication 1.
X = halogène.

4) A titre de médicaments utilisables en thérapeutique humaine ou vétérinaire, les composés selon les revendications 1 et 2 doués de propriétés antalgiques.

6) Les compositions pharmaceutiques contenant à titre de principe actif antalgique, un composé selon les revendications 1,2 et 4 utilisées en thérapeutique.

G) Les compositions selon la revendication 5 contenant d'autres principes actifs venant compléter l'action thérapeutique.

7) Les médicaments selon les revendications 4,5 et 6 contenant les excipients appropriés et les véhicules galéniques permettant l'administration par voie orale , locale, injectable ou rectale.

0099438

Office européen des brevets

# RAPPORT DE RECHERCHE EUROPEENNE

Numéro de la demande

EP 82 40 1345

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | FR-A-2 383 176 (P.FABRE)<br>*Revendications; exemple 5*<br>--- | 1,3-7 | C 07 D 253/06<br>C 07 D 401/12<br>A 61 K 31/53 |
| Y | FR-A-2 368 278 (P.FABRE)<br>*Revendications*<br>--- | 1,4-7 | |
| Y | EP-A-0 036 357 (P.FABRE)<br>*En entier*<br>----- | 1,3-7 | |

DOMAINES TECHNIQUES RECHERCHES (Int. Cl. 3)

C 07 D 253/00
C 07 D 401/00
A 61 K 31/00

Le présent rapport de recherche a été établi pour toutes les revendications

| Lieu de la recherche | Date d'achèvement de la recherche | Examinateur |
|---|---|---|
| LA HAYE | 04-03-1983 | NUYTS A.M.K.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant